# FASCICULE DE BREVET EUROPEEN

(11) **EP 3 254 043 B1**
(45) Date de publication et mention de la délivrance du brevet: **19.08.2020**
(21) Numéro de dépôt: 16705298.4
(22) Date de dépôt: 04.02.2016
(51) Int. Cl.: F27B 9/40, C21D 11/00, F27D 19/00, G01G 19/03, B21B 38/00, C21D 1/74, C21D 1/76, G01G 17/00, G01N 33/20

(54) **DISPOSITIF ET PROCEDE DE DETERMINATION DE LA PERTE AU FEU D'AU MOINS UNE PARTIE D'UN PRODUIT SIDERURGIQUE**
VORRICHTUNG UND VERFAHREN ZUR BESTIMMUNG DES GLÜHVERLUSTS VON MINDESTENS EINEM TEIL EINES EISEN- UND STAHLPRODUKTS
DEVICE AND METHOD FOR DETERMINING THE LOSS ON IGNITION OF AT LEAST PART OF AN IRON AND STEEL PRODUCT

(30) Priorité: 04.02.2015 FR 1550878
(43) Date de publication de la demande: 13.12.2017
(73) Titulaire: Fives Stein, 94700 Maisons Alfort (FR)
(72) Inventeur: CHAN, Yuen Yee, 94700 Maisons Alfort (FR); MAGALHAES, Jean-Luc, 78490 Boissy sans Avoir (FR)
(74) Mandataire: IP Trust
(86) Numéro de dépôt international: PCT/IB2016/050567
(87) Numéro de publication internationale: WO 2016/125096

(56) Documents cités:
- EP-A1- 0 767 353
- KR-A- 20120 032 870
- KR-A- 20120 096 997
- KR-A- 20130 134 328

## Description

### Domaine d'application

L'invention se rapporte à un procédé et dispositif de pilotage d'un four de réchauffage de produits sidérurgiques. Le dispositif et le procédé selon l'invention permettent de quantifier la perte au feu totale liée au réchauffage d'un produit dans le four, en déterminant la quantité de calamine qui est tombée dans le four et celle qui est tombée dans la décalamineuse. Ils permettent également de déterminer la quantité de calamine qui est restée accrochée au produit en sortie de décalamineuse.

Cette quantification est réalisée au moyen de capteurs électromagnétiques dont la résolution permet de mesurer avec précision l'épaisseur de la calamine.

### Art antérieur

L'invention appartient au domaine des fours de réchauffage de produits sidérurgiques.

Lors du réchauffage d'un semi-produit sidérurgique tel que billette, bloom ou brames dans un four de traitement thermique à feu nu, le produit s'oxyde en surface. La quantité, les types et qualités d'oxydes formés dépendent de la nature des aciers, de la composition chimique des fumées, des températures atteintes, des durées de séjour du produit dans les différentes zones du four, des différentes températures dans le four et de la courbe de chauffage du produit le long de sa traversée du four. Selon les valeurs de ces différents paramètres, la calamine qui se forme à la surface du produit est plus ou moins importante en qualité, en quantité et en comportement.

La calamine produite, par exemple comprise entre 0,5 et 1% du poids du produit enfourné, entraîne une perte de matière qui est éliminée avant l'entrée dans le laminoir et qui n'est donc pas transformée en produit fini (fil, profilé ou tôle), ce qui constitue une perte économique pour l'exploitant de l'installation.

Le chauffage de la partie de l'acier perdue sous forme de calamine engendre une perte d'énergie, une réduction du rendement global de l'installation et une augmentation du coût de production du produit fini.

La calamine produite peut se détacher dans le four lors de la traversée des produits pendant leur chauffage. L'accumulation de calamine dans les zones où elle se détache peut créer des dépôts dont l'importance perturbe le fonctionnement même du four et impose son arrêt pour nettoyage. Ce temps d'arrêt génère des pertes de production de l'installation et pénalise sa rentabilité moyenne durant l'ensemble de l'année.

La calamine qui a été formée à la surface du produit doit être évacuée avant laminage, généralement dans une décalamineuse projetant des jets d'eau à haute pression à la surface du produit pour détacher la calamine par choc thermique et action mécanique des flux d'eau projetés.

La calamine qui a été formée à la surface du produit peut rester adhérente, c'est-à-dire ne pas se détacher de cette surface, ni dans le four ni dans la décalamineuse, et suivre le produit dans les différents groupes de laminage. Cette situation peut conduire à des défauts à la surface du produit fini ou même provoquer des dégradations des rouleaux des groupes de laminage ou des casses de ces rouleaux.

On comprend ainsi que le procédé de réchauffage d'une ligne de laminage de produits sidérurgiques est fortement influencé par la formation de calamine qui peut avoir un impact direct sur la qualité du produit fini et/ou une influence importante sur le rendement du four, sur sa consommation et sur la durée de production entre deux arrêts pour maintenance.

Cette situation peut être rendue particulièrement difficile pour les fours de type produits plats qui réchauffent une grande quantité de produits de dimensions et de compositions d'aciers différents qui sont traités selon des courbes de chauffage ou des réglages de fours identiques, ce qui augmente les quantités de calamine produites et pénalise le bilan thermique et économique du four et de l'ensemble de la ligne de laminage.

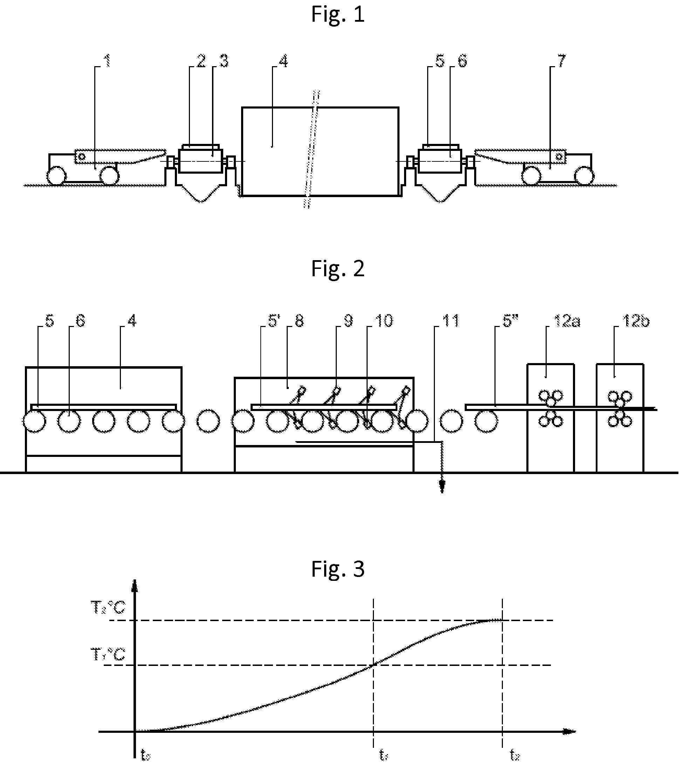

La quantité de calamine produite dépend du type de chauffage réalisé dans le four. La FIGURE 3 illustre un exemple de courbe de température d'un produit durant son temps total de réchauffage t₂ lors de sa traversée du four jusqu'à la température de défournement T₂. Ainsi, la FIGURE 3 présente en abscisse la longueur du four, ou de manière équivalente, le temps de séjour du produit dans le four qu'il traverse à vitesse constante. Durant un temps de séjour égal à t₂ - t₁, la température de la surface du produit se trouve au-dessus d'une température T₁, par exemple de 570 °C, à partir de laquelle cette surface s'oxyde sous l'action de l'oxygène présent dans la zone correspondante du four.

On comprend que le temps de séjour au-dessus de la température de formation de la calamine et le taux d'oxygène présent dans les fumées influencent directement la quantité de calamine formée ainsi que la nature éventuelle de cette calamine. Ces paramètres influencent également la quantité de calamine qui se dépose dans le four lors du réchauffage des produits, le décollement de la calamine dans l'installation de décalaminage et la quantité de calamine restante à la surface des produits qui peut provoquer des défauts de surface lors des opérations de laminage.

### Etat de l'art

La formation de calamine est considérée comme une composante inévitable du procédé de réchauffage de produits sidérurgiques et, même si elle est récupérée, ce recyclage masque des coûts de formation et de traitement de cette calamine.

La mesure de la formation de calamine lors du procédé de réchauffage est réalisée par l'étude d'échantillons déposés sur le produit et réchauffés avec lui. Cette méthode est donc essentiellement ponctuelle et ne se prête pas à un contrôle en continu du procédé de réchauffage pour chacun des produits traités lors de ce procédé.

Une autre méthode de mesure de la quantité de calamine produite consiste à peser la calamine récupérée à la décalamineuse, dans des bacs de décantation de l'eau de décalaminage ou dans des bacs de collecte évacués au pont roulant. Cette méthode n'est pas précise car on pèse de la calamine humide et elle ne permet pas de mesurer la calamine qui se dépose dans le four mais seulement celle qui est récupérée après la décalamineuse. Ainsi, il existe une approximation sur la mesure de la quantité de calamine produite. D'autre part, les durées entre pesées de la calamine sont longues, de l'ordre de plusieurs heures entre deux mesures. Enfin, les quantités de calamine pesées peuvent correspondre à plusieurs lots de produits réchauffés dans le four. Ainsi, la méthode est globale et donc approchée.

Les méthodes de mesure de la quantité de calamine produite selon l'état de l'art sont donc ponctuelles et approximatives. Elles ne peuvent assurer un contrôle en continu de la production de calamine dans le four pour agir en temps réel sur les paramètres de fonctionnement du four.

La calamine, quand elle est prise en compte par l'exploitant du four, est gérée suivant des réglages standards censés apporter une réponse moyenne à ce phénomène quelles que soient les dimensions des produits, des matériaux qui les composent et des cycles de chauffage réalisés.

En particulier, il est souvent réalisé des valeurs de réglages du four qui augmentent la quantité de calamine produite pour permettre de se situer dans une zone de confort de fonctionnement de l'installation pour laquelle la calamine se détache facilement du produit lors de son passage dans la décalamineuse par exemple, au détriment de la quantité de calamine produite et du coût total de formation, de collecte et de traitement de cette calamine produite.

Les fours fonctionnent avec plusieurs types de gaz, par exemple du gaz naturel, du gaz mixte (mélange de plusieurs gaz) ou du gaz de cokerie. Ces différents gaz produisent des compositions de fumées différentes dont les effets sur la formation de la calamine sur la surface du produit sont différents. Ces différences ne sont actuellement pas prises en compte dans le pilotage des fours.

On connaît les documents brevets KR 2013 0134328 qui divulgue un dispositif de détection de la présence de calamine, KR 2012 0032870 et KR 2012 0096997 qui propose un procédé de mesure de brame. On connaît également le document brevet EP 0 767 353 qui propose une Méthode pour égaliser la température d'un four à oxydation ambiante contrôlée et four pour la réaliser

Un but de l'invention est de proposer un procédé de pilotage d'un four de réchauffage de produits sidérurgiques remédiant à tout ou partie des inconvénients précités.

Un but de l'invention est de pallier tout ou partie des inconvénients de l'état de la technique, et/ou d'améliorer la souplesse et la simplicité de pilotage d'un four de réchauffage tout en conservant ou en améliorant la robustesse et le coût de ce pilotage, de la maintenance et/ou du fonctionnement des moyens par lesquels ce four de réchauffage est piloté.

### Exposé de l'invention

On atteint au moins l'un de ces buts avec un procédé de pilotage d'un four de réchauffage de produits sidérurgiques, comprenant :
- une détermination pour tout ou partie d'un produit de la quantité de calamine formée par un réchauffage de la partie de produit, cette détermination étant réalisée à partir de données mesurées relatives à la partie avant et après le réchauffage tel que décrit dans le procédé de la revendication 7,
- une correction de paramètres de fonctionnement du four en fonction de la quantité de calamine formée ainsi déterminée de manière à modifier la quantité de calamine formée par le réchauffage.

Ainsi, la mesure est réalisée à partir de données propres à ladite partie de produit.

La détermination étant réalisée à partir de données mesurées sur ladite partie, on simplifie la détermination de la calamine, on évite la mise en œuvre d'étapes de pose et dépose d'un échantillon et de moyens physiques et humains pour mettre en œuvre cette pose et dépose.

Bien entendu, la détermination de la quantité de calamine peut être réalisée pour tout le produit.

Bien entendu, les données mesurées après ledit réchauffage et relatives à ladite partie peuvent être mesurées après ledit réchauffage ou après la décalamineuse.

La détermination de la quantité de calamine à partir de données mesurées sur le produit avant et juste après le four, c'est-à-dire avant la décalamineuse, permet de déduire la quantité de calamine restée dans le four.

La détermination de la quantité de calamine à partir de données mesurées sur le produit avant et après la décalamineuse, permet de déduire la quantité de calamine produite au cours de l'ensemble du procédé.

On peut également optimiser les caractéristiques d'adhérence de la calamine sur le produit. Cette optimisation permet de réduire les défauts sur les produits laminés.

Avantageusement, la détermination de la quantité de calamine est réalisée de manière périodique ou continue.

Avantageusement, la correction des paramètres de fonctionnement du four est réalisée de manière périodique ou continue.

Contrairement à l'art antérieur, le procédé selon l'invention peut être mis en œuvre de manière aisée, sans perturber le processus classiquement utilisé de réchauffage, ni celui de laminage.

De préférence, une détermination de la quantité de calamine et/ou une correction de paramètres de fonctionnement du four est réalisée pour chacun des produits enfournés.

Il est ainsi possible de contrôler en continu la production de calamine.

La détermination de la quantité de calamine peut être réalisée de façon continue sur l'ensemble des produits entrant et sortant du four.

La correction d'un ou de plusieurs paramètres du four peut être réalisée en continu selon les informations collectées par les capteurs installés sur le four.

Ainsi, l'invention apporte une solution au contrôle en continu de la production de calamine dans le four de façon à en optimiser la quantité et la qualité produite pour qu'elle soit évacuée facilement de la surface des produits dans la décalamineuse et pour que cette quantité résiduelle arrivant jusqu'aux cages de laminoirs soit la plus faible possible.

Le procédé de pilotage selon l'invention peut comprendre une détermination de la part de calamine détachée, par exemple par le mouvement de longerons mobiles et fixes ou les supports des produits durant leur transfert sur toute la longueur du four.

Lorsque la part de calamine détachée ainsi déterminée est supérieure à un paramètre prédéterminé, par exemple lorsque cette part est jugée importante, le procédé de pilotage peut réaliser une modification des paramètres du four de manière à modifier le comportement de la calamine à la surface du produit, en particulier en augmenter l'adhérence sur la face inférieure du produit. Cette modification peut en particulier agir sur un paramètre tel que l'excès d'air, l'oxygène résiduel dans les fumées ou l'injection de vapeur d'eau ou par l'utilisation de combustibles différents dans diverses zones du four.

Les mesures réalisées sur une partie du produit sont effectuées sur cette même partie du produit avant et après le réchauffage.

Selon l'invention, les données mesurées sont obtenues par mesure de l'épaisseur du produit ou encore de ses dimensions.

Selon un premier aspect de l'invention, les données mesurées sont obtenues par mesure de l'une au moins des dimensions du produit, cette mesure étant réalisée par des capteurs électromagnétiques disposés avant et après le réchauffage afin de déterminer la quantité de calamine formée à la surface du produit avant la décalamineuse.

De préférence, les capteurs électromagnétiques sont orientés de manière à observer les faces inférieures et/ou supérieures du produit.

De préférence, les capteurs électromagnétiques sont positionnés de façon à observer deux parties d'une même surface du produit, une des parties de cette surface portant la calamine à mesurer, l'autre partie de cette surface ayant été nettoyée par exemple par décalaminage partiel.

Avantageusement, les capteurs électromagnétiques sont des capteurs à laser bleu, c'est-à-dire dont la longueur d'onde est comprise entre 445 et 405 nm. Les lasers bleus sont en effet bien adaptés au niveau de température des produits et à l'environnement dans lequel les capteurs se trouvent. Les essais menés avec des lasers rouges ont montré que ceux-ci sont moins précis lorsque le produit se situe à des niveaux de température élevés, par exemple de l'ordre de 1250°C. De même, les essais avec des capteurs à lumière blanche ont été moins concluants, les mesures étant perturbées par les rayonnements réfléchis sur le produit.

Les capteurs utilisés émettent un rayonnement électromagnétique qui balaie un secteur de l'espace sur un plan P avec un angle d'ouverture et à une fréquence définie. A un instant t, ils voient ainsi la surface d'une tranche transversale du produit.

Les capteurs sont avantageusement protégés par une isolation thermique. Ils sont par exemple placés dans des boîtiers calorifugés et climatisés dont une vitre en vitrocéramique permet le passage du rayonnement électromagnétique.

Selon un autre aspect de l'invention, une étape tenant compte de la dilatation du produit succède à une mesure de l'épaisseur du produit.

L'étape de détermination du procédé selon l'invention peut mettre en œuvre un procédé de détermination de la perte au feu d'au moins une partie d'un produit sidérurgique, dite produit, lors de son passage dans un four de réchauffage.

Selon deuxième aspect de l'invention, il est proposé un procédé de détermination de la perte au feu d'au moins une partie d'un produit sidérurgique, dite produit, lors de son passage dans un four de réchauffage.

Le procédé selon le deuxième aspect de l'invention met en œuvre un dispositif selon l'invention qui est décrit plus bas. La calamine tombée d'une surface balayée par un capteur est déterminée par l'analyse du relief de la surface obtenue par le capteur du dispositif.

De préférence, la calamine présente sur une surface balayée par un capteur est déterminée par l'analyse du relief de ladite surface obtenu par ledit capteur.

Selon troisième aspect de l'invention, il est proposé un procédé de détermination de la perte au feu d'au moins une partie d'un produit sidérurgique, dite produit, lors de son passage dans un four de réchauffage, éventuellement combinable avec tout autre aspect de l'invention ou l'un ou plusieurs de ses perfectionnements.

Le procédé selon le troisième aspect de l'invention met en œuvre un dispositif selon l'invention qui est décrit plus bas. Une quantité de calamine tombée dans la décalamineuse est déterminée par la différence de hauteur du produit entre l'amont et l'aval de la décalamineuse déterminée par le traitement des données fournies par les capteurs du dispositif.

De préférence, pour chaque ensemble de deux capteurs électromagnétiques, pour déterminer la hauteur du produit, on soustrait la hauteur entre la face inférieure du produit et la génératrice du rouleau déterminée par le capteur à la hauteur du produit déterminée par le capteur.

Selon un autre aspect de l'invention, éventuellement combinable avec tout ou partie des aspects précédents, la modification d'un paramètre de fonctionnement du four comprend une mise en œuvre d'une injection contrôlée de vapeur d'eau dans le four. Cette modification a pour objet d'agir sur la formation de la calamine à la surface des produits.

Selon un autre aspect de l'invention, éventuellement combinable avec tout ou partie des aspects précédents, la modification d'un paramètre de fonctionnement du four comprend une augmentation de la quantité d'air ou/et de comburant de combustion injecté dans le four. Cette modification a pour objet d'agir sur la formation de la calamine à la surface des produits.

Selon un autre aspect de l'invention, éventuellement combinable avec tout ou partie des aspects précédents, la modification d'un paramètre de fonctionnement du four comprend une mise en œuvre d'atmosphères particulières dans différentes zones du four, en particulier d'atmosphères présentant des taux d'oxygène contrôlés. On peut ainsi obtenir des valeurs résiduelles d'oxygène correspondants au degré d'oxydation recherché. Cette modification a pour objet de modifier les qualité et quantité de calamine produites.

Selon un autre aspect de l'invention, éventuellement combinable avec tout ou partie des aspects précédents, la quantité de produits dans le four est ajustée en fonction de la production désirée.

Selon un autre aspect de l'invention, éventuellement combinable avec tout ou partie des aspects précédents, la modification d'un paramètre de fonctionnement du four comprend une mise en œuvre de plusieurs types de combustibles pour alimenter les brûleurs du four et produire des atmosphères différentes. Cette modification a pour objet de réduire la quantité de calamine produite.

Selon un autre aspect de l'invention, éventuellement combinable avec tout ou partie des aspects précédents, un paramètre de fonctionnement du four comprend une mise en œuvre de courbes de chauffage du produit.

Selon un autre aspect de l'invention, éventuellement combinable avec tout ou partie des aspects précédents, le procédé selon l'invention comprend une optimisation de la quantité de métal perdu dans et hors du four pendant le processus de réchauffage du produit.

Selon un autre aspect de l'invention, il est proposé un dispositif de détermination de la perte au feu d'au moins une partie d'un produit sidérurgique, dite produit, lors de son passage dans un four de réchauffage situé en amont d'une décalamineuse, le produit circulant de préférence sur des tables à rouleaux, le dispositif comprenant un ensemble de capteurs électromagnétiques, lequel ensemble comprend :
- au moins un capteur électromagnétique dudit ensemble étant agencé pour balayer selon un plan de balayage au moins en partie la face inférieure du produit au voisinage de la sortie du four, ledit capteur électromagnétique étant orienté de sorte que ledit plan de balayage du rayonnement électromagnétique dudit capteur soit perpendiculaire à une direction de défilement du produit,
- un ensemble d'au moins deux capteurs électromagnétiques placés en amont de la décalamineuse et orientés de sorte que les plans de balayage de leurs rayonnements électromagnétiques soient sensiblement sur un même plan perpendiculaire à la direction de défilement de ladite au moins une partie du produit passant par la génératrice d'un rouleau d'une table à rouleaux, et un ensemble d'au moins deux capteurs électromagnétiques placés en aval de la décalamineuse et orienté de sorte que les plans de balayage de leurs rayonnements électromagnétiques soient sensiblement sur un même plan perpendiculaire à la direction de défilement du produit passant par la génératrice d'un rouleau d'une table à rouleaux, lesdits capteurs étant agencés pour déterminer la hauteur du produit en amont et en aval de la décalamineuse.

Selon un autre aspect de l'invention, éventuellement combinable avec tout ou partie des aspects précédents, les capteurs sont agencés pour balayer la face supérieure du produit et les capteurs sont agencés pour balayer une face latérale du produit.

Selon un autre aspect de l'invention, éventuellement combinable avec tout ou partie des aspects précédents, les plans de balayage des rayonnements électromagnétiques des capteurs sont inclinés d'un angle, noté *α*, par rapport à l'axe longitudinal des rouleaux des tables à rouleaux.

Selon un autre aspect de l'invention, il est proposé un dispositif de pilotage d'un four de réchauffage de produits sidérurgiques, le dispositif étant configuré pour mettre en œuvre un procédé de pilotage selon l'invention et comprenant :
- des moyens de détermination configurés pour déterminer pour une partie d'un produit une quantité de calamine formée par un réchauffage de ladite partie de produit, ces moyens étant mis en œuvre avant et après ledit réchauffage,
- des moyens de correction d'un paramètre de fonctionnement du four en fonction de la quantité de calamine formée ainsi déterminée, ces moyens de correction étant configurés de manière à réduire la quantité de calamine formée par le réchauffage.

Selon un autre aspect qui ne fait pas partie de la présente invention, il est proposé un produit programme de calculateur comprenant des instructions qui conduisent le dispositif selon l'invention à exécuter les étapes du procédé de pilotage selon l'invention.

Ainsi, l'invention permet d'optimiser en continu et pour chaque produit réchauffé ou de façon périodique sur une sélection de produits réchauffés le fonctionnement du four en mesurant la quantité de calamine formée durant le passage des produits dans le four et, à partir de cette indication de qualité et de quantité d'en déduire les réglages optimaux à appliquer au procédé de réchauffage afin de réduire la quantité de calamine et/ou d'en maîtriser la formation dans le four pour réduire les consommations d'énergie de l'installation ou réduire les problèmes de laminage des produits après leur réchauffage.

### Description des figures

D'autres particularités et avantages de l'invention apparaîtront à la lecture de la description détaillée de mises en œuvre et de modes de réalisation nullement limitatifs, au regard de figures annexées sur lesquelles :
- la FIGURE 1 présente une vue schématique d'une installation de réchauffage d'un produit sidérurgique ;
- la FIGURE 2 présente une vue schématique d'une installation de décalaminage et de laminage du produit réchauffé par l'installation de réchauffage ;
- La FIGURE 3 illustre un exemple de courbe de température du produit durant son temps de chauffage dans l'installation de réchauffage ;
- La FIGURE 4 illustre schématiquement l'implantation d'un capteur électromagnétique venant scruter la surface d'un produit selon l'invention;
- La FIGURE 5 illustre schématiquement l'implantation d'un capteur électromagnétique venant scruter la surface inférieure d'un produit selon l'invention;
- La FIGURE 6 illustre schématiquement l'implantation de capteurs venant mesurer la hauteur d'un produit selon l'invention;
- La FIGURE 7 illustre schématiquement l'implantation d'un capteur selon l'invention venant mesurer la distance entre le bord d'un produit et le rouleau sur lequel il repose ;
- La FIGURE 8 illustre schématiquement l'implantation d'un capteur selon l'invention venant réaliser une estimation de la hauteur d'un produit.

### Description de l'invention

Ces modes de réalisation n'étant nullement limitatifs, on pourra notamment réaliser des variantes de l'invention ne comprenant qu'une sélection de caractéristiques décrites par la suite, telles que décrites ou généralisées, isolées des autres caractéristiques décrites, si cette sélection de caractéristiques est suffisante pour conférer un avantage technique ou pour différencier l'invention par rapport à l'état de la technique.

La FIGURE 1 présente le principe d'une installation de laminage de produit sidérurgique. Une machine d'enfournement 1, par exemple à doigts, saisit un produit sidérurgique 2 amené par une table à rouleaux 3. La table à rouleaux 3 amène le produit 2 face à un four 4 de réchauffage de produits sidérurgiques. Le produit 2 saisit est placé par la machine d'enfournement 1 dans le four 4 sur des longerons de transfert (non représentés).

Lors de sa traversée du four, le produit 2 à enfourner dans le four se réchauffe progressivement selon une courbe de chauffage prédéterminée, par exemple pour être porté de la température ambiante externe au four 4 jusqu'à typiquement une température de défournement à la sortie du four comprise entre 1100 °C et 1300 °C.

Un produit réchauffé 5 est sorti du four par une machine à doigts 7 et placé sur une autre table à rouleaux 6 qui l'évacue vers un laminoir (non représenté).

La FIGURE 2 montre la table à rouleaux 6 d'évacuation du produit réchauffé 5 après sa sortie du four 4. Ce produit est déplacé par la table à rouleaux 6 vers une décalamineuse 8. Sur la FIGURE 2, le produit au sein de la décalamineuse 8 est numéroté 5'. Le produit 5' est exposé dans la décalamineuse 8 à des jets d'eau 9, 10 à haute pression. Les jets d'eau 9, 10 à haute pression sont respectivement orientés sur une partie supérieure et inférieure du produit 5'. Ces jets d'eau 9, 10 sont agencés pour décoller la calamine formée à la surface du produit 5' et évacuer la calamine décollée selon un circuit 11 vers des bacs de décantation (non représentés) pour sa récupération.

Après décalaminage par la décalamineuse 8, le produit est amené en entrée d'une lamineuse. Dans la lamineuse, le produit est référencé 5". Le produit 5" passe dans différentes sections de laminage 12a, 12b. Les sections de laminage 12a, 12b sont agencées pour obtenir un fil, profilé ou tôle souhaité à partir du produit 5".

Dans des installations selon l'état de l'art, la calamine récupérée dans le circuit 11 est pesée pour définir de façon globale la masse récupérée et la perte au feu, c'est-à-dire la quantité relative de calamine produite durant l'opération de réchauffage du produit.

Selon l'invention, un dispositif de mesure en continu de la calamine produite par le réchauffage est disposé à la sortie du four 4, éventuellement après la décalamineuse. Ce dispositif de mesure est agencé pour comparer la quantité de calamine à des limites fixées selon le mode de chauffage et à la nature de l'acier réchauffé dans le four.

Cette comparaison permet de déduire une efficacité de chauffe du four et de développer une stratégie corrective du chauffage apte à ramener la calamine produite dans les limites de quantité et de qualité souhaitées.

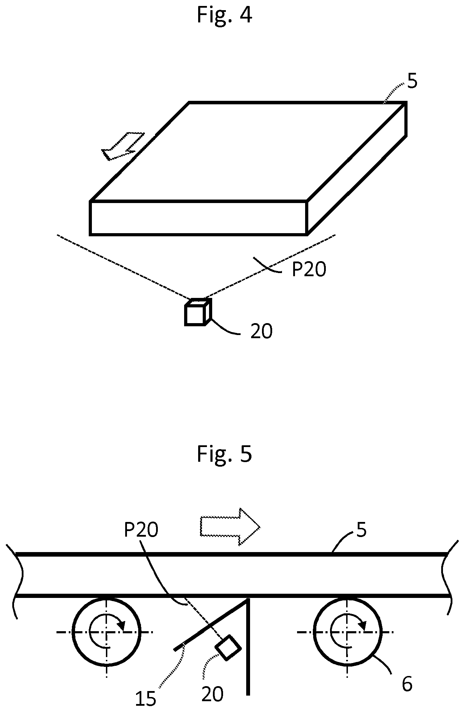

Les FIGURES 4 à 8 présentent des dispositifs de mesure en continu de la calamine par mesure de son épaisseur par le moyen de capteurs de mesure de distance optique.

Une mesure est réalisée par analyse optique sur la largeur du produit ainsi que sur la longueur du produit lors de son déplacement devant le capteur. Pour chaque point de la zone scrutée par un capteur, c'est-à-dire de la surface du produit vue par le capteur, une mesure de distance est réalisée avec une précision de l'ordre du micromètre qui permet la mesure de la hauteur réelle, c'est-à-dire de l'épaisseur du produit.

Il est ainsi aisé de calculer le volume du produit, donc son poids avant et après réchauffage avec, par différence, la quantité de calamine évacuée.

La mesure réalisée permet également de faire une évaluation de l'épaisseur de calamine formée et, ainsi, de réaliser une compensation de la masse de calamine qui s'est détachée du produit et qui est tombée dans le four et dans la décalamineuse. Il est également possible par le calcul de réaliser la compensation de la dilatation des produits. Ces calculs peuvent être réalisés avec des algorithmes physiques simples.

Sur la Figure 4, on peut voir schématiquement représenté un capteur électromagnétique 20 dont le rayonnement électromagnétique balaie la surface de la face inférieure d'un produit 5 en se déplaçant sur un plan P20 selon un angle d'ouverture. Sur cette figure, le produit 5 est représenté en coupe transversale.

L'éloignement du capteur par rapport au produit et l'angle d'ouverture du capteur permettent de couvrir toute la largeur du produit. Lorsque l'éloignement du produit et/ou l'angle d'ouverture du capteur ne permettent pas de couvrir toute la largeur du produit, plusieurs capteurs sont avantageusement utilisés pour couvrir toute la largeur du produit.

Cependant, pour limiter le coût de l'installation, il est possible de n'installer qu'un seul capteur et d'utiliser les données collectées par ce capteur pour les transposer sur la surface du produit non couverte par le capteur.

On considère ainsi, par approximation, que la quantité et les caractéristiques de la calamine sont les mêmes sur la surface couverte par le capteur et celle non couverte par le capteur.

Une partie de la quantité de calamine tombée dans le four 4 est ainsi déterminée par au moins un capteur 20 placé sous le produit 5, qui scrute la face inférieure de celui-ci.

Ledit capteur est placé en sortie de four et au plus près de celui-ci.

Le capteur réalise une cartographie du relief de la face inférieure du produit lors du défilement de celui-ci. L'analyse de la cartographie du relief de la surface du produit permet de déterminer la quantité de calamine tombée dans le four. En effet, les points hauts à la surface du produit correspondent aux endroits où la calamine est toujours présente sur le produit. A l'inverse, les points bas correspondent aux endroits à la surface du produit où la calamine s'est décrochée et est tombée dans le four.

L'analyse des données fournies par le capteur permet de déterminer d'éventuels points singuliers, par exemple un point sensiblement plus haut que la moyenne des points hauts. En ce point, il est probable que de la calamine s'est fortement décollée du produit mais est restée présente sur celui-ci. L'analyse statistique des données fournies par le capteur permet de prendre en compte ces points singuliers, par exemple afin de les écarter lors du traitement des données pour ne pas perturber la détermination de l'épaisseur de la calamine.

Le capteur 20 étant placé sous le produit, il est nécessaire d'éviter que de la calamine ne tombe sur celui-ci et vienne gêner son fonctionnement. Pour cela, un écran 15 incliné par rapport au niveau du sol est placé entre le produit 5 et le capteur 20.

Cet écran doit être sensiblement transparent pour le faisceau de sorte de ne pas dégrader la précision de la mesure. Il peut par exemple s'agir d'une plaque en vitrocéramique.

L'inclinaison de l'écran est choisie de sorte que la calamine qui tombe sur l'écran glisse et ne reste pas sur celui-ci.

Le capteur étant placé sous l'écran, il est incliné du même angle que l'écran de sorte d'éviter toute perturbation optique du laser lors de la traversée de l'écran.

Pour une détermination plus fine de la perte de calamine dans le four, un capteur est placé de chaque côté du produit en sortie de four. Tout comme le capteur placé au-dessus du produit, ces capteurs réalisent une cartographie du relief des faces latérales du produit lors du défilement de celui-ci afin de déterminer la quantité de calamine formée sur lesdites faces latérales qui est tombée dans le four.

Dans le cas où seul capteur est placé sur l'une des faces du produit, la quantité de calamine totale perdue par les deux faces du produit est estimée en comptant deux fois celle de la face du produit instrumentée.

Avantageusement selon l'invention, un capteur est également placé au-dessus du produit en sortie de four de sorte de réaliser une cartographie de la face supérieure du produit. Comme cette calamine reste majoritairement présente sur le produit en sortie de four, cette cartographie de la face supérieure n'est donc pas utilisée pour déterminer la quantité de calamine tombée dans le four. Cette cartographie permet par exemple de déceler une différence d'oxydation sur la face supérieure du produit ce qui peut être utile pour l'optimisation des paramètres de fonctionnement du four.

Sur la Figure 5, on peut voir représenté schématiquement un produit 5 en défilement sur une table à rouleaux 6 de sortie de four selon une vue latérale longitudinale.

Un capteur électromagnétique 20 est placé sous le produit. Son rayonnement électromagnétique balaie la surface de la face inférieure du produit en se déplaçant sur un plan P20.

Un écran incliné 15 est placé entre le produit 5 et le capteur 20. Cet écran permet d'éviter que de la calamine se dépose sur le capteur et ne gêne son fonctionnement.

Le capteur 20 est incliné du même angle que l'écran incliné 15 de sorte que le plan P20 de balayage du capteur est perpendiculaire à l'écran 15.

La quantité de calamine tombée dans la décalamineuse 8 est déterminée par deux ensembles de capteurs, le premier placé en amont de la décalamineuse, le second en aval de la décalamineuse.

Chaque ensemble de capteurs comprend au moins un premier capteur 30, 40 placé sur la face supérieure du produit et au moins un second capteur 31, 41 placé sur l'un des côtés du produit.

Les capteurs 30 et 31 sont placés en amont de la décalamineuse et les capteurs 40 et 41 sont placés en aval de la décalamineuse.

Nous ne décrirons par la suite que l'ensemble de capteurs 30 et 31 sachant que la disposition de ces capteurs est identique à celle des capteurs 40 et 41.

Le capteur 30 placé au-dessus du produit est disposé à la verticale d'un rouleau 14 de la table à rouleaux sur laquelle circulent les produits. Il permet de mesurer la distance entre la face supérieure du produit 5 et la génératrice supérieure du rouleau 14. Pour un produit reposant parfaitement sur le rouleau 14 support, cette distance correspond à la hauteur du produit.

Le capteur est placé de sorte que son champ de mesure couvre au moins en partie la face supérieure du produit et au moins une partie de la génératrice supérieure dudit rouleau.

Le capteur est avantageusement incliné d'un angle alpha par rapport à l'axe longitudinal dudit rouleau, par exemple d'un angle de 5°. Cette inclinaison permet de garantir que le faisceau du capteur couvre en au moins un point 18 la génératrice supérieure du rouleau. En effet, si le capteur était disposé avec son champ de mesure parallèle à l'axe du rouleau, il serait nécessaire d'avoir un alignement vertical parfait du capteur par rapport au rouleau de sorte que le capteur voit la génératrice supérieure du rouleau et non une génératrice placée sur un plan inférieur. Ce capteur permet également de mesurer le relief de la face supérieure du produit couverte par son champ de mesure.

Le capteur placé sur le côté du produit est disposé sur le même plan vertical que le capteur placé au-dessus du produit, c'est-à-dire au niveau de la génératrice du même rouleau support. Lorsque le capteur placé au-dessus du produit ne couvre pas les deux côtés du rouleau support situés de part et d'autre du produit, le capteur placé sur le côté du produit est situé sur le côté du rouleau dont le capteur situé en face supérieure du produit voit la génératrice.

Le capteur placé sur le côté du produit permet de corriger la hauteur du produit mesurée par le capteur placé en face supérieure lorsque le produit ne repose pas exactement sur le rouleau support. En effet, pour un produit déformé qui ne reposerait pas sur la génératrice du rouleau, la hauteur du produit déterminée par le capteur supérieur correspondrait à la somme de la hauteur réelle du produit prendre et de celle du jeu entre la face inférieure du produit et la génératrice du rouleau.

La combinaison de ces deux capteurs permet une mesure précise de la hauteur du produit. La comparaison de la hauteur du produit mesurée par le premier ensemble de capteurs disposé en amont de la décalamineuse et celle mesurée par le second ensemble de capteurs disposé en aval de la décalamineuse permet de déterminer la perte de hauteur du produit dans la décalamineuse. Cette perte de hauteur correspond à l'essentiel de la calamine tombée dans la décalamineuse.

Les capteurs placés sur le côté du produit permettent également de réaliser une cartographie du relief de la face du produit qu'ils scrutent.

L'analyse de la cartographie du relief de la face du produit permet de déterminer la quantité de calamine tombée dans le four, pour le capteur placé en amont de la décalamineuse, et celle tombée dans la décalamineuse pour le capteur placé en aval de la décalamineuse. En effet, les points hauts à la surface du produit correspondent aux endroits où la calamine est toujours présente sur le produit. A l'inverse, les points bas correspondent aux endroits à la surface du produit où la calamine s'est décrochée et est tombée dans le four.

Lorsque des capteurs latéraux ne sont placés que sur l'une des faces du produit, la quantité de calamine totale perdue par les deux faces du produit est estimée en comptant deux fois celle de la face du produit instrumentée.

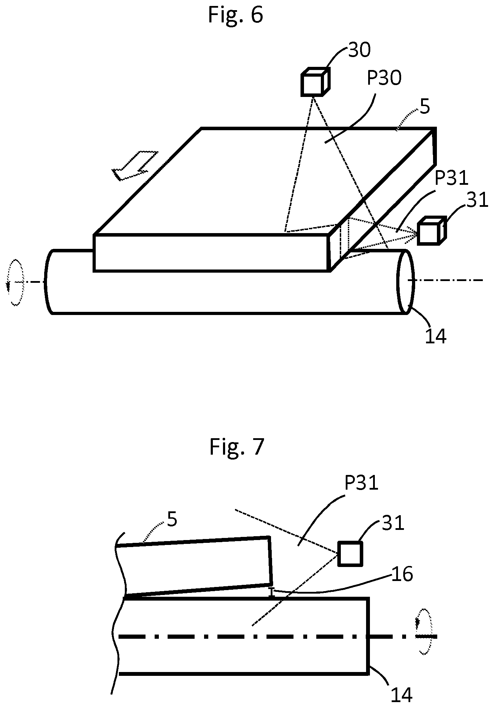

Sur la Figure 6, on peut voir représenté en vue transversale un produit 5 en défilement sur une table à rouleaux.

Un capteur électromagnétique 30 est placé au-dessus du produit et scrute une portion de la face supérieure du produit ainsi qu'une portion du rouleau 14 de la table à rouleau situé à la verticale du capteur.

Le plan P30 sur lequel se déplace le faisceau du capteur est perpendiculaire au produit et sensiblement parallèle à l'axe du rouleau 14 tout en étant incliné d'un angle alpha par rapport à cet axe.

Le capteur 30 permet de réaliser une première estimation de la hauteur du produit 5 par mesure de la distance entre la face supérieure et produit et le point haut de la génératrice du rouleau 14.

Un capteur électromagnétique 31 est placé sur le côté du produit et scrute la face latérale du produit ainsi qu'une portion du rouleau 14.

Le plan P31 sur lequel se déplace le faisceau du capteur 31 est perpendiculaire au rouleau et passe par l'axe du rouleau. La génératrice supérieure du rouleau 14 se trouve ainsi sur le plan P31.

Le capteur 31 permet d'analyser le relief de la face latérale du produit et de mesurer un éventuel espace entre le bord du produit 5 et la génératrice du rouleau 6.

Sur la Figure 7, on peut voir représenté en vue transversale un agrandissement de la figure 6 au niveau du capteur 31 montrant un produit 5 déformé dont le bord latéral ne repose pas sur le rouleau 14. Le capteur 31 permet ainsi de mesurer la hauteur 16 de l'espace entre le bord du produit 5 et la génératrice du rouleau 14. Cette hauteur est soustraite de la hauteur du produit déterminé par le capteur 30 pour obtenir la hauteur réelle du produit.

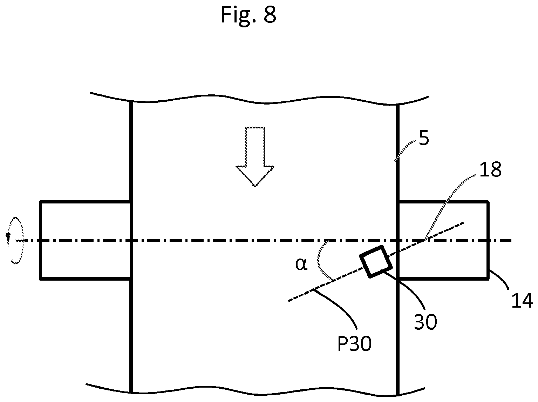

Sur la Figure 8, on peut voir schématiquement représenté en vue de dessus un produit 5 en défilement sur une table à rouleaux dont un rouleau 14 est représenté.

Un capteur électromagnétique 30 est placé au-dessus du produit et scrute une portion de la face supérieure du produit ainsi qu'une portion du rouleau 14.

Le plan P30 sur lequel se déplace le faisceau du capteur est perpendiculaire au produit et sensiblement parallèle à l'axe du rouleau 14 tout en étant incliné d'un angle alpha par rapport à cet axe. Cette inclinaison du capteur permet de garantir que le plan P30 traverse la génératrice supérieure du rouleau 14 en un point 18. Le capteur 30 permet ainsi de réaliser une première estimation de la hauteur du produit 5 par mesure de la distance entre la face supérieure et produit et ce point haut 18 de la génératrice du rouleau.

Ces différents dispositifs selon les FIGURES 4 à 8 peuvent être mis en œuvre à différentes étapes du processus de fabrication, en particulier pour mettre en évidence une différence dans les dimensions des produits ou dans leur poids significatif de la production de calamine en quantité ou de son comportement.

On peut ainsi mettre en évidence la quantité qui peut se déposer dans le four durant le réchauffage ou après le four lors de la prise du produit par la machine de défournement ou à chaque étape du procédé après le four, par exemple lors du transfert du produit sur les tables à rouleaux, dans la décalamineuse ou dans les différentes unités du laminoir.

L'homme du métier sait en effet placer de tels capteurs aux alentours du four. Le capteur est protégé dans une boîte refroidie à l'eau et vise au travers d'un verre de visée balayé à l'air froid qui le maintient en température malgré le rayonnement qu'il reçoit du four ou du produit.

En particulier, on comprend l'intérêt de mettre en place un dispositif de mesure du produit avant son enfournement dans le four ainsi qu'un autre après son défournement ou après la décalamineuse afin d'obtenir par différence entre ces mesures une image de la quantité de calamine produite et de son comportement. Il est également possible de réaliser plusieurs mesures, par exemple avant le four, en sortie de four et après la décalamineuse pour mieux évaluer les différentes étapes de la vie de la calamine.

Le dispositif décrit par les FIGURES 4 à 8 peut être installé en entrée de four pour définir un modèle volumique du produit à son enfournement, il peut être installé en sortie du four ou en sortie de la décalamineuse pour réaliser un modèle volumique du produit après réchauffage et décalaminage.

La comparaison des modèles permet de tirer des enseignements sur le résultat du chauffage. Cet enseignement peut être utilisé en particulier pour agir sur les consignes du four afin d'en modifier la courbe de chauffage et/ou le pilotage des brûleurs et/ou l'atmosphère dans l'enceinte du four et en particulier l'excès d'air et/ou une éventuelle injection de vapeur d'eau dans certaines zones du four et/ou faire fonctionner le four avec des zones réductrices et des zones oxydantes et/ou modifier les paramètres de réglage de la décalamineuse tels que pression d'eau, nombre de rampes de décalaminage utilisées, vitesse d'avance du produit.

La saisie de ces informations sur le produit, avant et après réchauffage, est traitée par un calculateur selon des modèles physiques simples ou élaborés, par exemple pour tenir compte du comportement de la calamine, d'une évaluation de la partie du poids de calamine se déposant dans le four lors du réchauffage, d'une évaluation de la calamine formée à la surface supérieure du produit et à sa surface inférieure. On peut ainsi prendre en compte la chute d'une partie de la calamine formée sur la face inférieure du produit lors de son transfert sur les longerons du four ou sur les tables à rouleaux d'évacuation ou encore une évaluation de la partie résiduelle de calamine à la surface du produit après le décalaminage. Un produit programme de calculateur ne faisant pas partie de l'invention est proposé comprenant des instructions de code de programme pour l'exécution des étapes du procédé selon l'une quelconque des revendications selon l'invention lorsque le programme est exécuté sur un calculateur.

On peut également envisager la mise en œuvre d'un produit programme d'ordinateur, par exemple de type logique floue ou auto-adaptatif, pour analyser en continu la formation de calamine sur le produit pour valider l'action opérée sur la conduite du four ou évaluer les évolutions de la calamine (en quantité et en qualité) au fil du temps selon les modifications du procédé opérées.

On voit que par le moyen de la mesure en continu de la quantité de calamine formée à la surface du produit et du système de conduite du four par calculateur, il est possible d'adapter en continu les paramètres de conduite du four selon une stratégie ou des objectifs prédéfinis, par exemple réduire la quantité de calamine fournie, stabiliser la quantité de calamine produite à une valeur prédéfinie en fonction de la nature du produit à traiter et de son procédé de traitement, de modifier la quantité de calamine produite afin d'obtenir une qualité de calamine adaptée au procédé, par exemple pour ses caractéristiques d'évacuation dans la décalamineuse.

Ce procédé de pilotage du four en continu selon la mesure de la calamine produite permet d'optimiser le procédé complet de laminage et d'optimiser la consommation énergétique en réduisant la quantité de calamine produite.

Bien sûr, l'invention n'est pas limitée aux exemples qui viennent d'être décrits et de nombreux aménagements peuvent être apportés à ces exemples sans sortir du cadre des revendications. De plus, les différentes caractéristiques, formes, variantes et modes de réalisation de l'invention peuvent être associés les uns avec les autres selon diverses combinaisons dans la mesure où ils ne sont pas incompatibles ou exclusifs les uns des autres.

### Nomenclature

- 1: machine d'enfournement
- 2: produit sidérurgique
- 3: table à rouleaux d'entrée de four
- 4: four de réchauffage
- 5: produit sidérurgique
- 5': produit dans la décalamineuse
- 5": produit dans une lamineuse
- 6: table à rouleaux de sortie de four
- 7: machine de défournement
- 8: décalamineuse
- 9: jet d'eau supérieur haute pression
- 10: jet d'eau inférieur haute pression
- 11: circuit d'évacuation
- 12a, 12 b: sections de laminage
- 14: rouleau support
- 15: écran incliné
- 16: Distance entre le bord du produit et un rouleau support
- 18: Intersection entre un plan P30 et la génératrice supérieure d'un rouleau
- 20: capteur électromagnétique balayant la face inférieure d'un produit
- 30: capteur électromagnétique balayant la face supérieure d'un produit en amont de la décalamineuse
- 31: capteur électromagnétique balayant une face latérale d'un produit en amont de la décalamineuse
- 40: capteur électromagnétique balayant la face supérieure d'un produit en aval de la décalamineuse
- 41: capteur électromagnétique balayant une face latérale d'un produit en aval de la décalamineuse
- P20: plan de balayage du laser d'un capteur 20
- P30: plan de balayage du laser d'un capteur 30
- P40: plan de balayage du laser d'un capteur 40
- P41: plan de balayage du laser d'un capteur 41
- P42: plan de balayage du laser d'un capteur 42

## Revendications

1. Dispositif de détermination de la perte au feu d'au moins une partie d'un produit sidérurgique (5), dite produit, lors de son passage dans un four (4) de réchauffage situé en amont d'une décalamineuse (8), le produit circulant de préférence sur des tables à rouleaux (3, 6),
**caractérisé en ce que** ledit dispositif comprenant un ensemble de capteurs électromagnétiques (20, 30, 31, 40, 41), lequel ensemble comprend :
. au moins un capteur électromagnétique (20) dudit ensemble étant agencé pour balayer selon un plan de balayage au moins en partie la face inférieure (50) du produit (5) au voisinage de la sortie du four (4), ledit capteur électromagnétique étant orienté de sorte que ledit plan (P20) de balayage du rayonnement électromagnétique dudit capteur soit perpendiculaire à une direction de défilement du produit,
. un ensemble d'au moins deux capteurs électromagnétiques (30, 31) placés en amont de la décalamineuse (8) et orientés de sorte que les plans (P30, P31) de balayage de leurs rayonnements électromagnétiques soient sensiblement sur un même plan (P32) perpendiculaire à la direction de défilement de ladite au moins une partie du produit passant par la génératrice d'un rouleau d'une table à rouleaux (3), et un ensemble d'au moins deux capteurs électromagnétiques (40, 41) placés en aval de la décalamineuse (8) et orienté de sorte que les plans (P40, P41) de balayage de leurs rayonnements électromagnétiques soient sensiblement sur un même plan (P42) perpendiculaire à la direction de défilement du produit passant par la génératrice d'un rouleau d'une table à rouleaux (6), lesdits capteurs (30, 31, 40, 41) étant agencés pour déterminer la hauteur du produit en amont et en aval de la décalamineuse.

2. Dispositif selon la revendication 1, dans lequel les capteurs (30, 40) sont agencés pour balayer la face supérieure (51) du produit (5) et les capteurs (31, 41) sont agencés pour balayer une face latérale (52, 53) du produit (5).

3. Dispositif selon l'une ou l'autre des revendications précédentes, dans lequel les plans (P30, P40) de balayage des rayonnements électromagnétiques des capteurs (30, 40) sont inclinés d'un angle, noté *α*, par rapport à l'axe longitudinal des rouleaux des tables à rouleaux (3, 6).

4. Dispositif selon l'une quelconque des revendications précédentes, dans lequel les capteurs sont protégés par une isolation thermique.

5. Dispositif selon la revendication précédente, dans lequel l'isolation thermique présente une vitre en vitrocéramique.

6. Dispositif selon l'une quelconque des revendications précédentes, dans lequel les capteurs électromagnétiques sont des capteurs dont la longueur d'onde est comprise entre 445 et 405 nm.

7. Procédé de détermination de la perte au feu d'au moins une partie d'un produit sidérurgique, dite produit, lors de son passage dans un four (4) de réchauffage mettant en œuvre un dispositif tel que décrit aux revendications précédentes, dans lequel la calamine tombée d'une surface balayée par un capteur (20, 31, 41) est déterminée par l'analyse de la cartographie du relief de ladite surface obtenue par le capteur (20, 31, 41).

8. Procédé selon la revendication précédente, dans lequel la calamine présente sur une surface balayée par un capteur (20, 31, 41) est déterminée par l'analyse du relief de ladite surface obtenu par ledit capteur (20, 31, 41).

9. Procédé de détermination de la perte au feu d'au moins une partie d'un produit sidérurgique (5), dite produit, lors de son passage dans un four (4) de réchauffage mettant en œuvre un dispositif tel que décrit aux revendications 1 à 6, dans lequel une quantité de calamine tombée dans la décalamineuse (8) est déterminée par la différence de hauteur du produit (5) entre l'amont et l'aval de la décalamineuse déterminée par le traitement des données fournies par les capteurs (30, 31, 40, 41).

10. Procédé selon la revendication précédente, dans lequel, pour chaque ensemble de deux capteurs électromagnétiques (30, 31), (40, 41), pour déterminer la hauteur du produit (5), on soustrait la hauteur entre la face inférieure du produit et la génératrice du rouleau déterminée par le capteur (31, 41) à la hauteur du produit (5) déterminée par le capteur (30, 40).

11. Procédé de pilotage d'un four (4) de réchauffage de produits sidérurgiques (5), comprenant :
- une détermination pour au moins une partie d'un produit (5) d'une quantité de calamine formée par un réchauffage de ladite partie de produit, cette détermination étant réalisée par la mise en œuvre du procédé décrit aux revendications 7 à 10,
- une correction de paramètres de fonctionnement du four en fonction de la quantité de calamine formée ainsi déterminée, comprenant l'une au moins des étapes suivantes :
∘ mise en œuvre d'une injection contrôlée de vapeur d'eau dans le four,
∘ augmentation de la quantité d'air ou/et de comburant de combustion injecté dans le four,
∘ mise en œuvre d'atmosphères particulières dans différentes zones du four, en particulier d'atmosphères présentant des taux d'oxygène contrôlés,
∘ mise en œuvre de plusieurs types de combustibles pour alimenter les brûleurs du four et produire des atmosphères différentes en vue de réduire la quantité de calamine produite,
∘ mise en œuvre de courbes de chauffage du produit.

12. Procédé de pilotage selon la revendication précédente, dans lequel les étapes de détermination de la quantité de calamine et/ou de correction de paramètre de fonctionnement du four sont réalisées de manière périodique ou en continu.

## Patentansprüche

1. Vorrichtung zur Bestimmung des Glühverlusts mindestens eines Teils eines als Produkt bezeichneten Stahlprodukts (5) während seines Durchlaufs durch einen Nachwärmofen (4), der sich vor einer Entzunderungsmaschine (8) befindet, wobei das Produkt vorzugsweise auf Rollgängen (3, 6) zirkuliert, **dadurch gekennzeichnet, dass** die Vorrichtung einen Satz elektromagnetischer Sensoren (20, 30, 31, 40, 41) umfasst, wobei der Satz umfasst:
. mindestens einen elektromagnetischen Sensor (20) des Satzes, der so angeordnet ist, dass er entlang einer Abtastebene mindestens teilweise die Unterseite (50) des Produkts (5) in der Nähe des Auslasses des Ofens (4) abtastet, wobei der elektromagnetische Sensor so ausgerichtet ist, dass die Abtastebene (P20) der elektromagnetischen Strahlung des Sensors senkrecht zu einer Laufrichtung des Produkts ist,
. einen Satz von mindestens zwei elektromagnetischen Sensoren (30, 31), die vor der Entzunderungsmaschine (8) angeordnet und so ausgerichtet sind, dass die Abtastebenen (P30, P31) ihrer elektromagnetischen Strahlung im Wesentlichen auf derselben Ebene (P32) senkrecht zur Laufrichtung des mindestens einen Teils des Produkts liegen, der durch die Mantellinie einer Rolle einer Rollbahn (3) läuft, und einen Satz von mindestens zwei elektromagnetischen Sensoren (40, 41) die der Entzunderungsmaschine (8) nachgelagert angeordnet und so ausgerichtet sind, dass die Abtastebenen (P40, P41) ihrer elektromagnetischen Strahlung im Wesentlichen auf derselben Ebene (P42) senkrecht zur Laufrichtung des Produkts liegen, das durch die Mantellinie einer Rolle einer Rollbahn (6) läuft, wobei die Sensoren (30, 31, 40, 41) so angeordnet sind, dass sie die Höhe des Produkts bestimmen, das der Entzunderungsmaschine vor- und nachgelagert ist.

2. Vorrichtung nach Anspruch 1, wobei die Sensoren (30, 40) angeordnet sind, um die obere Fläche (51) des Produkts (5) abzutasten, und die Sensoren (31, 41) angeordnet sind, um eine Seitenfläche (52, 53) des Produkts (5) abzutasten.

3. Vorrichtung nach einem der vorhergehenden Ansprüche, bei der die Ebenen (P30, P40) zum Abtasten der elektromagnetischen Strahlung der Sensoren (30, 40) in einem Winkel, notiert α, in Bezug auf die Längsachse der Rollen der Rollbahnen (3, 6) geneigt sind.

4. Vorrichtung nach einem der vorhergehenden Ansprüche, bei der die Sensoren durch Wärmedämmung geschützt sind.

5. Vorrichtung nach dem vorhergehenden Anspruch, bei der die Wärmedämmung eine Glaskeramikscheibe aufweist.

6. Vorrichtung nach einem der vorhergehenden Ansprüche, bei der die elektromagnetischen Sensoren Sensoren einer Wellenlänge zwischen 445 und 405 nm sind.

7. Verfahren zur Bestimmung des Glühverlusts mindestens eines Teils eines als Produkt bezeichneten Stahlprodukts während seines Durchlaufs durch einen Nachwärmofen (4) unter Verwendung einer Vorrichtung nach den vorhergehenden Ansprüchen, bei dem der Zunder, der von einer von einem Sensor (20, 31, 41) abgetasteten Oberfläche herabfällt, durch Analyse der vom Sensor (20, 31, 41) erhaltenen Abbildung des Reliefs der Oberfläche bestimmt wird.

8. Verfahren nach dem vorhergehenden Anspruch, bei dem der auf einer von einem Sensor (20, 31, 41) abgetasteten Oberfläche vorhandene Zunder bestimmt wird, indem das von dem Sensor (20, 31, 41) erhaltene Relief der Oberfläche analysiert wird.

9. Verfahren zur Bestimmung des Glühverlusts mindestens eines Teils eines als Stahlprodukt (5) bezeichneten Produkts während seines Durchlaufs durch einen Nachwärmofen (4) unter Verwendung einer Vorrichtung nach den Ansprüchen 1 bis 6, bei dem eine in die Entzunderungsmaschine (8) gefallene Zundermenge durch den Höhenunterschied des Produkts (5) zwischen der der Entzunderungsmaschine vor- und nachgelagerten Seite bestimmt wird, der durch die Verarbeitung der von den Sensoren (30, 31, 40, 41) gelieferten Daten bestimmt wird.

10. Verfahren nach dem vorhergehenden Anspruch, bei dem für jeden Satz von zwei elektromagnetischen Sensoren (30, 31), (40, 41) zur Bestimmung der Höhe des Produkts (5) die Höhe zwischen der Unterseite des Produkts und der durch den Sensor (31, 41) bestimmten Mantellinie der Rolle von der durch den Sensor (30, 40) bestimmten Höhe des Produkts (5) subtrahiert wird.

11. Verfahren zur Steuerung eines Nachwärmofens (4) für Stahlprodukte (5), umfassend:
- eine Bestimmung für mindestens einen Teil eines Produkts (5) einer durch Nachwärmen dieses Teils des Produkts gebildeten Zundermenge, wobei diese Bestimmung nach dem in den Ansprüchen 7 bis 10 beschriebenen Verfahren durchgeführt wird,
- eine Korrektur der Betriebsparameter des Ofens in Abhängigkeit von der auf diese Weise bestimmten Menge des gebildeten Zunders, umfassend mindestens einen der folgenden Schritte:
∘ Durchführung einer kontrollierten Dampfeinspritzung in den Ofen,
∘ Erhöhung der Menge der in den Ofen eingeblasenen Luft und/oder des Verbrennungsoxidationsmittels,
∘ Herstellung bestimmter Atmosphären in verschiedenen Bereichen des Ofens, insbesondere Atmosphären mit kontrolliertem Sauerstoffgehalt,
∘ Einsatz verschiedener Arten von Brennstoff zur Beschickung der Ofenbrenner und zur Erzeugung unterschiedlicher Atmosphären, um die Menge des erzeugten Zunders zu reduzieren,
∘ Herstellung von Produkterwärmungskurven.

12. Steuerungsverfahren nach dem vorhergehenden Anspruch, bei dem die Schritte der Bestimmung der Zundermenge und/oder der Korrektur des Betriebsparameters des Ofens regelmäßig oder durchgehend durchgeführt werden.

## Claims

1. A device for determining the scale loss of at least one part of a steel product (5), referred to as product, during the passage thereof through a reheating furnace (4) located upstream of a descaler (8), the product preferably moving on roller tables (3, 6), **characterized in that** said device comprises a set of electromagnetic sensors (20, 30, 31, 40, 41), which set comprises:
• at least one electromagnetic sensor (20) of said set being arranged in order to scan, along a scanning plane, at least in part, the lower face (50) of the product (5) in the vicinity of the outlet of the furnace (4), said electromagnetic sensor being oriented so that said scanning plane (P20) of the electromagnetic radiation of said sensor is perpendicular to a run direction of the product,
• a set of at least two electromagnetic sensors (30, 31) placed upstream of the descaler (8) and oriented so that the scanning planes (P30, P31) of the electromagnetic radiations thereof are substantially on one and the same plane (P32) perpendicular to the run direction of said at least one part of the product passing through the generatrix of a roller of a roller table (3), and a set of at least two electromagnetic sensors (40, 41) placed downstream of the descaler (8) and oriented so that the scanning planes (P40, P41) of the electromagnetic radiations thereof are substantially on one and the same plane (P42) perpendicular to the run direction of the product passing through the generatrix of a roller of a roller table (6), said sensors (30, 31, 40, 41) being arranged in order to determine the height of the product upstream and downstream of the descaler

2. The device as claimed in claim 1, wherein the sensors (30, 40) are arranged in order to scan the upper face (51) of the product (5) and the sensors (31, 41) are arranged in order to scan a side face (52, 53) of the product (5).

3. The device as claimed in any previous claims, wherein the scanning planes (P30, P40) of the electromagnetic radiations of the sensors (30, 40) are inclined at an angle, denoted α, with respect to the longitudinal axis of the rollers of the roller tables (3, 6).

4. Device according to any of the preceding claims in which the sensors are protected by thermal insulation

5. Device according to the preceding claim, in which the thermal insulation has a glass-ceramic pane.

6. Device according to any of the preceding claims, wherein the electromagnetic sensors are sensors whose wavelength is between 445 and 405 nm.

7. A process for determining the scale loss of at least one part of a steel product, referred to as product, during the passage thereof through a reheating furnace (4) using a device as described in any of the preceding claims, wherein the oxide scale fallen from a surface scanned by a sensor (20, 31, 41) is determined by analysis of the relief of said surface obtained by the sensor (20, 31, 41).

8. The process as claimed in the preceding claim, wherein the oxide scale present on a surface scanned by a sensor (20, 31, 41) is determined by analysis of the relief of said surface obtained by said sensor (20, 31, 41).

9. A process for determining the scale loss of at least one part of a steel product (5), referred to as product, during the passage thereof through a reheating furnace (4) using a device as described in claims 1 to 6, wherein an amount of oxide scale fallen into the descaler (8) is determined by the height difference of the product (5) between upstream and downstream of the descaler determined by the processing of the data provided by the sensors (30, 31, 40, 41).

10. The process as claimed in the preceding claim, wherein, for each set of two electromagnetic sensors (30, 31), (40, 41), in order to determine the height of the product (5), the height between the lower face of the product and the generatrix of the roller determined by the sensor (31, 41) is subtracted from the height of the product (5) determined by the sensor (30, 40).

11. A process for controlling a furnace (4) for reheating semi-finished steel products (5), comprising:
• a determination, for at least one part of a product (5), of an amount of oxide scale formed on the products by a reheating of said product part, this determination being carried out by the use of the process described in claims 7 to 10,
• a correction of operating parameters of the furnace as a function of the amount of oxide scale thus determined, comprising at least the following steps:
∘ a use of a controlled injection of steam into the furnace,
∘ an increase of the amount of combustion air and/or oxidant injected into the furnace,
∘ a use of particular atmospheres in various zones of the furnace, in particular of atmospheres having controlled oxygen contents,
∘ a use of several types of fuels for supplying the burners of the furnace and producing different atmospheres with a view to reduce the amount of oxide scale,
∘ a use of product heating curves.

12. The process as claimed in the preceding claim, wherein the step of determination of the oxide scale and/or the step of correction of operating parameters of the furnace are carried out periodically or continuously.
